# EUROPEAN PATENT SPECIFICATION

(11) **EP 1 511 530 B1**
(45) Date of publication and mention of the grant of the patent: **29.06.2011**
(21) Application number: 02788536.7
(22) Date of filing: 28.10.2002
(51) Int. Cl.: A61M 11/06

(54) **APPARATUS FOR NEBULISING A LIQUID, IN PARTICULAR FOR AEROSOL THERAPY**
VORRICHTUNG ZUM VERNEBELN EINER FLÜSSIGKEIT, INSBESONDERE ZUR AEROSOLTHERAPIE
APPAREIL SERVANT A NEBULISER UN LIQUIDE, EN PARTICULIER POUR THERAPIE PAR AEROSOL

(30) Priority: 28.05.2002 IT PR20020026
(43) Date of publication of application: 09.03.2005
(73) Proprietor: Koninklijke Philips Electronics N.V., 5621 BA Eindhoven (NL)
(72) Inventor: TROMBI, Andrea, I-43100 Parma (IT)
(74) Representative: Damen, Daniel Martijn
(86) International application number: PCT/IT2002/000681
(87) International publication number: WO 2003/099361

(56) References cited:
- EP-A- 0 626 180
- DE-C- 759 110
- US-A- 5 584 285
- US-A- 6 044 841

## Description

### TECHNICAL FIELD AND BACKGROUND ART.

The present invention relates to an apparatus for nebulising a liquid, in particular for aerosol therapy.

The apparatus comprises a nebulising ampoule provided with at least an opening for drawing and/or expelling air from/to the environment and is provided with a mouthpiece to dispense a nebulised medical product. The apparatus further comprises a valve for regulating a flow of air entering and/or exiting the ampoule, said valve being positioned in correspondence with the aforesaid opening.

As is well known, apparatuses for nebulising are used in particular in the field of aerosol therapy, i.e. of the therapeutic treatment of symptoms of the respiratory track, such as asthmatic or bronchial symptoms. Said therapeutic system provides for the generation of an aerosol, i.e. of a dispersion or nebulisation of appropriate medical liquids that act through the inhalation of the medical liquid itself. An example of nebulising apparatus is disclosed in US 5,584,285.

Such apparatuses are widely used, especially in the case of paediatric therapies, and are provided in different formats able to meet different users' requirements. More specifically, nebulising apparatuses can also be constructed in portable formats, so that the user can have the necessary medicine available at any time, especially in the case of ailments entailing frequent or unpredictable respiratory crises, such as asthmatic ailments.

Pneumatic nebulising apparatuses also exist, so defined because they comprise a compressor that aspirates air from the environment and sends it to a nebulising ampoule containing the medical liquid.

The compressor is generally housed in a rigid case, made of instance of plastic material, which incorporates the inlets and outlets of the aspiration and delivery conduits that come from the compressor itself. In use, mainly with apparatuses for home use, the rigid case containing the compressor is usually set down on a plane whilst the nebulising ampoule is located in proximity to the user's face and is connected to the inlet of the delivery conduit by means of a flexible pipeline.

The compressor can comprise a header incorporating both the aspiration conduit and the delivery conduit, interfacing directly with the exterior by means of intakes obtained directly on the header itself and destined to be adapted to the profile of the rigid containment case.

Some pneumatic nebulisation apparatuses are provided with a so-called supplementary, or secondary, channel, provided with an inlet through which ambient air enters by Venturi effect and because of the aspiration provided by the user during inspiration.

The flow of air of the secondary channel allows a better nebulisation of the medical product, in terms of quantity and quality of the generated spray. During the expiration phase, the air breathed out by the user is expelled from the apparatus by means of an outlet.

Normally, both the inlet of the secondary channel and the outlet are provided with a valve, able to move between an opened position and a closed position to guarantee that the flow of air inside the apparatus is correctly directed, both during inspiration and during expiration. In particular, said valves are usually made of highly deformable plastic material and are actuated directly by the flow of air that impacts thereon.

A possible known embodiment of said valves provides for the use of a rubber tab that has a first end fastened in correspondence with a mouth of a conduit and a second end free, in such a way as to cover the mouth itself and to be able to be lifted directly by a flow of air.

In accordance with a second type of prior art, valves for nebulisation apparatuses exist which are constituted by a ring whereto are peripherally welded a plurality of reeds, in such a way that a free end thereof (opposite to the welded one) can move under the direct actuation of the flow of air that traverses the valve. The ring provided with the reeds is inserted inside a pair of flanges, usually made of plastic material, mutually coupled by means of interference.

The valve types for nebulisation apparatuses briefly described above have some important drawbacks.

First of all, such valves, although made of a deformable material, are at times hard to operate and to open them and/or close them a flow of air with sufficient pressure is required. This has repercussions on the user, since (s)he sets the air aspiration and expulsion pressure during inspiration and expiration..

An additional drawback is represented by the fact that the aforementioned valves do not assure an effective closure, especially those that are normally closed in the resting configuration. In addition to compromising the correct operation of the nebulisation apparatus, this drawback entails inevitable wastage of medical product. A prior art valve is disclosed in DE 759 110 C.

### DISCLOSURE OF INVENTION.

An aim of the present invention is to eliminate the aforesaid drawbacks by making available an apparatus for nebulising a liquid, in particular for aerosol therapy, which is provided with valves that oppose a minimum resistance to opening and/or closing during inspiration and/or expiration by a user.

Another aim of the present invention is to propose a nebulising apparatus that is provided with valves which are normally closed in resting configuration and which assure a perfect seal, in order to limit wastage of medical product and assure the proper operation of the apparatus.

Said aims are fully achieved by the apparatus for nebulising a liquid, in particular for aerosol therapy, of the present invention, which is characterised by the content of the claims set out below and in particular in that the valve for regulating a flow of air is of the type comprising:
- a shutter able to move between an operative blocking configuration, corresponding to an obstruction of the opening, and an operative configuration consenting to the passage of the flow of air;
- a ring connected to the shutter to anchor it to a tubular portion of the ampoule, said tubular portion being positioned in correspondence with the opening;
- a plurality of deformable connecting elements between the ring and the shutter to allow the shutter to move from said operative blocking configuration to said operative consent configuration and vice versa, said movement being directly caused by the flow of air entering and/or exiting the ampoule.

### BEST MODE FOR CARRYING OUT OF THE INVENTION.

This and other aims will become more readily apparent from the description that follows of a preferred embodiment illustrated, purely by way of non limiting example, in the accompanying drawing table, in which:
- Figure 1 shows a partially sectioned front view of an apparatus for nebulising a liquid according to the present invention;
- Figure 2 shows a top view of the apparatus shown in Figure 1;
- Figure 3 shows a perspective view of the valve for regulating the flow of air.

With reference to the figures, the apparatus for nebulising a liquid in accordance with the present invention is globally indicated with the number 1 and comprises a nebuliser ampoule 2 provided with a mouthpiece 3 for dispensing a nebulised medical product directly into a user's oral cavity. The nebuliser ampoule 2 is provided with a first opening 4 for aspirating air from the environment, during the inspiration phase, and with a second opening 5 for expelling air into the environment, during the expiration phase. The apparatus 1 comprises a valve 6 for regulating a flow of air entering the nebuliser ampoule 2. This valve is positioned in correspondence with the aforesaid opening 4 necessary to aspirate air from the environment and it is preferably associated to a so-called supplementary, or secondary, channel 7 of the nebuliser ampoule.

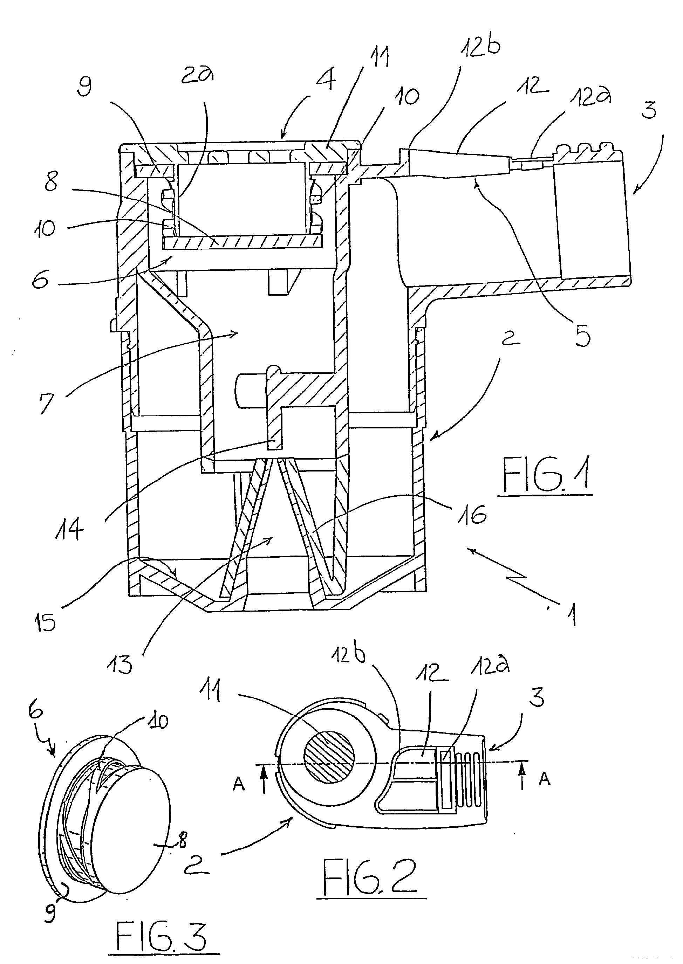

The flow regulating valve 6 is of the type comprising a shutter 8 able to move between an operative blocking configuration corresponding to an obstruction of the opening 4 (as shown in Figure 1) and an operative configuration corresponding to an obstruction of the opening 4 (as shown in Figure 1) and an operative configuration (not shown herein) of consent to the passage of the flow of air.

The shutter 8 is anchored to a tubular portion 2a of the nebuliser ampoule 2 by means of a ring 9 connected to the shutter by means of a plurality of deformable elements 10 which elements, given their deformability, allow the shutter 8 to move from the operative blocking configuration to the operative consent configuration and vice versa. In particular, said movement is directly caused by the vacuum generated on the shutter 8 by the user. In the illustrated embodiment, the deformable elements 10 are spiral shaped and have a first end fastened peripherally to the shutter 8 and a second end fastened to the ring 9.

The shutter 8, the ring 9 and the deformable elements 10 are preferably obtained in a single body and are made of polymeric material, typically rubber.

The apparatus 1 further comprises a protective element 11, holed and positioned in correspondence with the opening 4, to avoid introducing foreign bodies in the nebuliser ampoule 2, preventing any damage to the valve 6. In the illustrated embodiment, the apparatus 1 further comprises a second shutter 12 to cover the second opening 5, necessary to expel into the environment air exhaled by a user. In the illustrated embodiment, the second shutter 12 is constituted by a deformable reed-like body, typically a rubber tab, having an end 12a fastened in correspondence with the opening 5 and an end 12b free to move away from said opening to uncover it at least partially and allow air to escape.

In accordance with an embodiment variation not shown herein, it is possible to use a second valve 6 coupled to an opening necessary to expel the air exhaled by the user.

The apparatus 1 comprises a compressor (not shown), which sends air to the nebuliser ampoule 2 by means of a primary delivery channel 13 which preferably has conical shape. As soon as the air coming from the compressor impacts against an activator element 14, inside the ampoule such a turbulence is generated as to create a sufficient vacuum to aspirate the medical liquid through a series of channels (not shown herein) obtained directly on a substantially conical element 16, positioned in correspondence with the primary channel 13.

The supplementary conduit 7 enables to increase the nebulisation of medical products, and also allows a coarse selection of the particles present in the spray. In particular, particles of greater size are forced to settle on a bottom 15 of the nebuliser ampoule 2 and therefore only the particles having the optimal dimensions to render the aerosol therapy more effective come out of the dispensing mouthpiece 3.

During inspiration, the vacuum generated inside the ampoule 2 draws air in from the environment, forcing the shutter 8 to move away from the opening 4. At the end of the inspiration phase, the shutter moves to the resting configuration, i.e. to obstruct the opening 4, reducing spray formation. In this way, during the expiration phase, the wastage of medical product that exits the opening 5, borne by the air exhaled by the user, is limited. The exhaled air exits thanks to the rising of the second shutter 12 which uncovers, at least partially, the second opening 5 of the nebuliser ampoule. The rising of the second shutter 12 is made possible by the pressure exerted by the air exhaled by the user.

The invention achieves important advantages.

First of all, the presence of the valve 6 allows considerably to reduce the effort required from the user during the inspiration phase and possibly also during the expiration phase, if an additional valve 6 is adopted in correspondence with the opening 5, necessary to expel the air exhaled by the user. Moreover, with a reduced effort on the user's part, it is possible to generate such a displacement of the shutter 8 as to maximise the area of the passage section of the air flow.

Another advantage is provided by the fact that the presence of the deformable elements 10 enables the valve to operate in immediate fashion, opening it and closing it in very short time intervals. This allows to optimise the operation of the apparatus 1, reducing in particular the wastage of medical product.

Advantageously, such an apparatus is simple and economical to construct.

## Claims

1. Apparatus (1) for nebulising a liquid, in particular for aerosol therapy, of the type comprising:
a nebulising ampoule (2) provided with at least an opening (4;5) for aspirating and/or expelling air from/to the environment and with a mouthpiece (3) for dispensing a nebulisation of medical product;
a valve (6) for regulating a flow of air into and/or out of the ampoule (2), said valve (6) being positioned in correspondence with said opening (4;5) and comprising a shutter (8) able to move between an operative blocking configuration corresponding to an obstruction of said opening (4;5) and an operative configuration of consent to the passage of the flow of air,
a ring (9) connected to the shutter (8) to anchor it to a tubular portion (2a) of the ampoule (2), said tubular portion (2a) being positioned in correspondence and with the opening (4;5) and,
a plurality of deformable connecting elements (10) between the ring (9) and
the shutter (8) to allow said shutter (8) to move from said operative blocking configuration to said operative configuration of consent and vice versa, said movement being directly caused by an inspiration and/or expiration phase by a user,
wherein the deformable elements (10) are spiral shaped and have a first end fastened peripherally to the shutter (8) and a second end fastened to the ring (9).

2. Apparatus as claimed in claim 1, **characterised in that** it comprises a holed protective element (11) positioned in correspondence with the opening (4;5) to prevent the introduction of foreign bodies into the ampoule (2), preventing any damage to the valve (6).

3. Apparatus as claimed in claim 1, **characterised in that** it comprises a second shutter (12) to cover an opening (5) of the ampoule (2) necessary to expel to the environment air exhaled by a user, said second shutter (12) being a deformable reed-like body having an end (12a) fastened in correspondence with the opening (5) and an end (12b) that is free to move away from the opening (5) to uncover it at least partially and allow the escape of air.

4. Apparatus as claimed in claim 1, **characterised in that** the valve (6) is associated with a so-called supplementary, or secondary, channel (7) of the nebuliser ampoule (2).

## Patentansprüche

1. Vorrichtung (1) zum Vernebeln einer Flüssigkeit, insbesondere zur Aerosoltherapie, eines Typs, der Folgendes umfasst:
eine Vernebelungsampulle (2) mit mindestens einer Öffnung (4; 5) zum Ansaugen und/oder Ausstoßen von Luft aus der Umgebung bzw. in die Umgebung und mit einem Mundstück (3) zum Abgeben einer Vernebelung eines medizinischen Produkts;
ein Ventil (6) zum Regulieren eines Luftstroms in die bzw. aus der Ampulle (2), wobei das genannte Ventil (6) in Übereinstimmung mit der genannten Öffnung (4; 5) angeordnet ist; und
eine Verschlussklappe (8), die in der Lage ist, sich zwischen einer betriebsfähigen sperrenden Konfiguration, welche einer Blockierung der genannten Öffnung (4; 5) entspricht, und einer betriebsfähigen Konfiguration des Zulassens des Durchflusses des Luftstroms zu bewegen;
einen Ring (9), der mit der Verschlussklappe (8) verbunden ist, um sie an einem röhrenförmigen Abschnitt (2a) der Ampulle (2) zu verankern, wobei der genannte röhrenförmige Abschnitt (2a) in Übereinstimmung mit der Öffnung (4; 5) angeordnet ist; und
eine Vielzahl von verformbaren Verbindungselementen (10) zwischen dem Ring (9) und der Verschlussklappe (8), um der genannten Verschlussklappe (8) zu erlauben, sich von der genannten betriebsfähigen sperrenden Konfiguration in die genannte betriebsfähige Konfiguration des Zulassens und umgekehrt zu bewegen, wobei die genannte Bewegung direkt durch eine Einatmungs- und/oder Ausatmungssphase durch einen Benutzer verursacht wird,
wobei die verformbaren Elemente (10) spiralförmig sind und ein erstes Ende haben, das peripher an der genannten Verschlussklappe (8) befestigt ist, und ein zweites Ende haben, das an dem Ring (9) befestigt ist.

2. Vorrichtung nach Anspruch 1, **dadurch gekennzeichnet, dass** sie ein ausgehöhltes Schutzelement (11) umfasst, welches in Übereinstimmung mit der Öffnung (4; 5) angeordnet ist, um das Einführen von Fremdkörpern in die Ampulle (2) zu verhindern und **dadurch** eine Beschädigung des Ventils (6) zu verhindern.

3. Vorrichtung nach Anspruch 1, **dadurch gekennzeichnet, dass** sie eine zweite Verschlussklappe (12) umfasst, um eine Öffnung (5) der Ampulle (2) abzudecken, die erforderlich ist, um durch einen Benutzer ausgeatmete Luft in die Umgebung auszustoßen, wobei die genannte zweite Verschlussklappe (12) ein verformbarer blatt-ähnlicher Körper ist, dessen eines Ende (12a) in Übereinstimmung mit der Öffnung (5) befestigt ist und dessen anderes Ende (12b) frei ist, um sich von der Öffnung (5) weg zu bewegen, um diese zumindest teilweise freizugeben, damit Luft entweichen kann.

4. Vorrichtung nach Anspruch 1, **dadurch gekennzeichnet, dass** das Ventil (6) einem so genannten ergänzenden oder sekundären Kanal (7) der Verneblerampulle (2) zugeordnet ist.

## Revendications

1. Appareil (1) de nébulisation d'un liquide, en particulier pour thérapie par aérosol, du type comprenant :
une ampoule de nébulisation (2) dotée d'au moins une ouverture (4 ; 5) pour aspirer et/ou expulser l'air depuis/vers l'environnement et d'un embout (3) destiné à distribuer un produit médical nébulisé ;
une valve (6) destinée à réguler un flux d'air dans et/ou hors de l'ampoule (2), ladite valve (6) étant positionnée en correspondance avec ladite ouverture (4 ; 5) ; et comprenant :
un obturateur (8) capable de se déplacer entre une configuration opérationnelle de blocage correspondant à une obstruction de ladite ouverture (4 ; 5) et une configuration opérationnelle permettant le passage du flux d'air ;
une bague (9) reliée à l'obturateur (8) pour l'ancrer dans une partie tubulaire (2a) de l'ampoule (2), ladite partie tubulaire (2a) étant positionnée en correspondance avec l'ouverture (4 ; 5) ; et
une pluralité d'éléments de raccordement déformables (10) entre la bague (9) et l'obturateur (8) de manière à permettre à l'obturateur (8) de se déplacer de ladite configuration opérationnelle de blocage à ladite configuration opérationnelle de passage et inversement, ledit déplacement étant directement provoqué par une phase d'inspiration et/ou d'expiration d'un utilisateur,
dans lequel les éléments déformables (10) sont en forme de spirale et ont une première extrémité fixée de manière périphérique à l'obturateur (8) et une seconde extrémité fixée à la bague (9).

2. Appareil selon la revendication 1, **caractérisé en ce qu'**il comprend un élément protecteur perforé (11) positionné en correspondance avec l'ouverture (4 ; 5) pour empêcher l'introduction de corps étrangers dans l'ampoule (2) en empêchant toute détérioration de la valve (6).

3. Appareil selon la revendication 1, **caractérisé en ce qu'**il comprend un second obturateur (12) pour recouvrir une ouverture (5) de l'ampoule (2) nécessaire pour expulser dans l'environnement l'air exhalé par un utilisateur, ledit second obturateur (12) étant un corps déformable en forme de languette ayant une extrémité (12a) fixée en correspondance avec l'ouverture (5) et une extrémité (12b) libre de s'écarter de l'ouverture (5) pour la découvrir au moins partiellement et laisser l'air s'échapper.

4. Appareil selon la revendication 1, **caractérisé en ce que** la valve (6) est associée à un canal supplémentaire ou secondaire (7) de l'ampoule de nébulisation (2).
